# FASCICULE DE BREVET EUROPEEN

(11) **EP 1 529 522 B1**
(45) Date de publication et mention de la délivrance du brevet: **26.08.2009**
(21) Numéro de dépôt: 04292288.0
(22) Date de dépôt: 23.09.2004
(51) Int. Cl.: A61K 8/67, A61Q 19/08, A61P 17/02, A61K 31/455, A61K 31/375

(54) **Composition de peeling comprenant de la vitamine B3 et de la vitamine C**
Vitamine B3 und Vitamine C enthaltende Peelingzusammensetzung
Peeling composition comprising vitamin B3 and vitamin C

(30) Priorité: 29.10.2003 FR 0350742
(43) Date de publication de la demande: 11.05.2005
(73) Titulaire: L'ORÉAL, 75008 Paris (FR)
(72) Inventeur: Sore, Gabrielle, 75012 Paris (FR); Hansenne, Isabelle, Westfield New Jersey 07090 (US)
(74) Mandataire: Le Coupanec, Pascale A.M.P.

(56) Documents cités:
- WO-A-00/71094
- WO-A-98/34610
- WO-A-98/46206
- GB-A- 2 210 789
- IT-A- PN 950 010
- US-A- 5 411 741
- US-A- 6 071 888
- PATENT ABSTRACTS OF JAPAN vol. 0183, no. 85 (C-1227), 20 juillet 1994 (1994-07-20) & JP 6 107531 A (KAO CORP), 19 avril 1994 (1994-04-19)

## Description

La présente invention se rapporte à un procédé cosmétique de peeling comprenant l'application topique sur la peau d'une composition adaptée à une application topique sur la peau et comprenant, dans un milieu physiologiquement acceptable : (a) de 10 à 70% en poids d'au moins un composé choisi parmi : l'acide nicotinique, les esters d'acide nicotinique et la nicotinamide, et (b) de 10 à 70% en poids d'au moins un composé choisi parmi : l'acide ascorbique et ses précurseurs choisis parmi le palmitate d'ascorbyle, le stéarate d'ascorbyle, l'ascorbyl phosphate de magnésium, l'ascorbyl phosphate de sodium, l'ascorbyl sulfate disodique, l'acide sulfinyl-L-ascorbique et l'acide glucopyranosyl-L-ascorbique, par rapport au poids total de la composition.

Elle se rapporte également à l'utilisation de la composition précitée pour fabriquer une préparation dermatologique destinée à réaliser un peeling chimique superficiel par application topique sur la peau, en vue d'atténuer les rides et ridules et/ou les taches pigmentaires et/ou les cicatrices telles que les marques d'acné ou de varicelle.

L'acide nicotinique et la nicotinamide sont des co-facteurs enzymatiques regroupés sous le terme de "vitamine B3" ou "vitamine PP". Outre son intérêt nutritionnel, mis à profit dans le domaine alimentaire, la vitamine B3 a notamment des propriétés vasodilatatrices, kératolytiques, anti-séborrhéiques, anti-inflammatoires et de régulation de la différenciation épidermique et de la prolifération dermique, ainsi que des propriétés dépigmentantes, qui en font un actif de choix dans le domaine cosmétique.

De son côté, l'acide ascorbique, ou vitamine C, possède de nombreuses vertus utilisables en cosmétique, et notamment des propriétés anti-radicalaires, dépigmentantes, anti-âge -par stimulation de la biosynthèse de différentes formes de collagène, de la laminine, des protéoglycanes et des céramides épidermiques et inhibition des métalloprotéinases matricielles-.

La vitamine C par ailleurs été décrite comme constituant possible, en tant qu'agent blanchissant, d'une composition exfoliante susceptible d'inclure par ailleurs de la nicotinamide comme inhibiteur de mélanogénèse (US-5,874,463) et incluant, comme constituant essentiel, au moins un composé choisi parmi l'acide salicylique, l'acide rétinoïque, l'acide pyruvique, le résorcinol et l'acide glycolique. Dans ce même document, elle est utilisée en tant que constituant minoritaire d'un peeling superficiel à base d'acide salicylique.

Les peelings chimiques sont un moyen bien connu pour améliorer l'aspect de surface de la peau, en particulier pour atténuer des défauts de pigmentation tels que les lentigo actiniques ou les marques d'acné ou de varicelle, ou pour lisser les irrégularités de la texture de la peau, en particulier les rides et ridules, en provoquant une destruction limitée de l'épiderme et des couches superficielles du derme.

Le document GB-A-2 210 789 décrit une composition topique pour peeling comprenant de l'acide nicotinique comme agent vasodilatateur et un peroxyde comme agent kératolityque et desquamant.

Le document WO 00/71094 décrit une composition exfoliante à base d'acide ascorbique.

Les proportions utilisées dans ces documents sont nettement inférieures aux proportions en vitamines C et B3 utilisées dans la présente demande.

Bien que les compositions décrites dans le brevet US-5,874,463 aient pu donner des résultats satisfaisants, il n'en reste pas moins qu'elles ne sont pas dénuées d'effets secondaires. Ainsi, les peelings à l'acide salicylique peuvent donner lieu à des cas de salicylisme en cas de surdosage ou d'application prolongée. En outre, l'acide rétinoïque est associé à des risques de tératogenèse et il augmente la sensibilité de la peau vis-à-vis des UV.

Il reste donc le besoin de disposer de compositions de peeling qui soient efficaces tout en étant bien tolérées.

Il est maintenant apparu à la Demanderesse que les vitamines B3 et C, ou leurs précurseurs, associées à fortes concentrations dans une composition topique, pouvaient permettre la réalisation de peelings chimiques efficaces et bien tolérés, sans qu'il soit nécessaire d'y ajouter d'autres actifs exfoliants.

Il a certes été déjà décrit dans le brevet US-6,071,888 des compositions, susceptibles d'être utilisées par voie topique, comprenant de 1 à 20% en poids d'acide nicotinique (ou ses précurseurs) et de 10 à 30% en poids d'acide ascorbique. Ces compositions sont destinées au traitement des mélanomes par stimulation de la réponse immunitaire. Il n'est pas prévu que ces compositions soient appliquées sur des peaux présentant des imperfections cutanées visibles ou tactiles, puis rincées pour réaliser un peeling destiné à atténuer ces imperfections.

La présente invention a donc pour objet un procédé cosmétique de peeling comprenant l'application topique sur la peau d'une composition adaptée à une application topique sur la peau et comprenant, dans un milieu physiologiquement acceptable : (a) de 10 à 70% en poids d'au moins un composé choisi parmi : l'acide nicotinique, les esters d'acide nicotinique et la nicotinamide, et (b) de 10 à 70% en poids d'au moins un composé choisi parmi : l'acide ascorbique et ses précurseurs, par rapport au poids total de la composition.

Elle se rapporte également à l'utilisation de cette composition pour fabriquer une préparation dermatologique destinée à réaliser un peeling chimique superficiel par application topique sur la peau, en vue d'atténuer les rides et ridules et/ou les taches pigmentaires et/ou les cicatrices telles que les marques d'acné ou de varicelle.

Le premier constituant de la composition selon l'invention est choisi parmi l'acide nocotinique, ses esters et la nicotinamide. Comme esters d'acide nicotinique utilisables dans la présente invention, on peut citer les alkyl esters d'acide nicotinique et d'un alcool en C₁-C₂₀, choisi par exemple parmi les alcools méthylique, éthylique, butylique, hexylique, octylique, décylique, dodécylique et myristylique. La nicotinamide est toutefois préférée pour une utilisation dans la présente invention.

Le second constituant de cette composition est choisi parmi l'acide ascorbique et ses précurseurs. Les précurseurs d'acide ascorbique, sont choisis parmi le palmitate d'ascorbyle le stéarate d'ascorbyle, l'ascorbyl phosphate de magnésium, l'ascorbyl phosphate de sodium, l'ascorbyl sulfate disodique, l'acide sulfinyl-L-ascorbique et l'acide glucopyranosyl-L-ascorbique. L'acide ascorbique est préféré pour une utilisation dans la présente invention.

La quantité de chacun des composés (a) et (b) selon l'invention représente de 10 à 70% du poids de la composition. De préférence, la quantité de composé (a) varie de 10 à 50 % du poids de la composition et, mieux, de 10 à 40 % du poids de la composition. La quantité de composé (b) varie de préférence de 10 à 50 % du poids de la composition et, mieux, de 10 à 40 % du poids de la composition. L'homme du métier saura ajuster ces quantités en fonction de la profondeur du peeling qui doit être réalisé, c'est-à-dire des couches de l'épiderme ou du derme qui doivent être enlevées.

La composition selon l'invention est adaptée à une application topique sur la peau et comprend donc généralement un milieu physiologiquement acceptable, c'est-à-dire compatible avec la peau et éventuellement avec ses phanères.

Elle peut se présenter sous toutes les formes galéniques classiquement utilisées pour une application topique, pour autant qu'elle s'élimine facilement par rinçage, et notamment sous forme de gel aqueux, de solution aqueuse ou hydroalcoolique. Elle peut aussi, par ajout d'une phase grasse ou huileuse, se présenter sous forme de dispersion du type lotion ou d'émulsion de consistance liquide ou semi-liquide, obtenue de préférence par dispersion d'une phase grasse dans une phase aqueuse (H/E). Ces compositions sont préparées selon les méthodes usuelles.

Selon un mode préféré de réalisation de l'invention, la composition renferme une phase aqueuse continue.

Lorsque la composition est sous forme d'émulsion, la proportion de la phase huileuse de l'émulsion peut aller par exemple de 1 à 30 % en poids, et de préférence de 5 à 20 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Comme matières grasses utilisables dans l'invention, on peut utiliser les huiles et notamment les huiles minérales (huile de vaseline), les huiles d'origine végétale (huile d'avocat, huile de soja), les huiles de synthèse (perhydrosqualène), les huiles siliconées (cyclométhicone) et les huiles fluorées (perfluoropolyéthers).

Comme émulsionnants et coémulsionnants utilisables dans l'invention, on peut citer par exemple les esters d'acide gras et de polyéthylène glycol tels que le stéarate de PEG-100, le stéarate de PEG-50 et le stéarate de PEG-40 ; les esters d'acide gras et de polyol tels que le stéarate de glycéryle, le tristéarate de sorbitane et les stéarates de sorbitane oxyéthylénés disponibles sous les dénominations commerciales Tween® 20 ou Tween® 60, par exemple ; et leurs mélanges.

La composition selon l'invention peut également contenir les adjuvants habituels dans les domaines cosmétique et dermatologique, tels que les épaississants, les actifs, les conservateurs, les solvants, et les charges. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse ou dans la phase aqueuse. Ces adjuvants, ainsi que leurs concentrations, doivent être tels qu'ils ne nuisent pas aux propriétés des composés selon l'invention.

Comme épaississants, on peut citer en particulier : la gomme de xanthane, un homo- ou copolymère d'acide acrylique éventuellement réticulé, un polyacrylamide, un homo- ou copolymère d'acide acrylamido méthylpropane sulfonique, et les dérivés de cellulose dont l'hydroxypropyl cellulose.

La composition renferme avantageusement au moins un composé choisi parmi les alcools et les polyols comprenant de 1 à 3 atomes de carbone. Comme exemples de tels composés, on peut citer l'éthanol, l'isopropanol, le glycérol et le propylène glycol.

En outre, selon une forme d'exécution préférée de l'invention, la composition ne contient pas d'acide rétinoïque ni d'acide glycolique ni de résorcinol, ni d'acide pyruvique ni d'acide salicylique, ni de chitosane.

Comme indiqué précédemment, cette composition est destinée à être utilisée pour réaliser un peeling chimique superficiel visant à atténuer les rides et ridules et/ou les taches pigmentaires et/ou les cicatrices. La composition est de préférence utilisée sur des personnes présentant de l'acné et/ou des rides et/ou des cicatrices et/ou des défauts de pigmentation tels que des mélasmas et des lentigos séniles ou actiniques. Elle peut être appliquée sur le visage et/ou le cou et/ou le décolleté et/ou les mains et/ou le dos.

Le procédé selon l'invention comprend de préférence les étapes consistant à :
(a) appliquer topiquement sur une zone de peau humaine une composition telle que définie dans l'une quelconque des revendications 1 à 6,
(b) laisser la composition au contact de la peau pendant une durée comprise entre 5 mn et 6 heures, et
(c) éliminer la composition par rinçage.

De façon préférée, la composition est laissée au contact de la peau, dans l'étape (b), pendant une durée comprise entre 5 minutes et 30 minutes.

La composition utilisée selon l'invention peut être appliquée par tout moyen permettant une répartition uniforme et notamment à l'aide d'un coton, d'une tige, d'un pinceau, d'une gaze, d'une spatule ou d'un tampon, ou encore par pulvérisation, et peut être éliminée par rinçage à l'eau ou à l'aide d'un détergent doux après être restée au contact de la peau.

Pour optimiser ses effets, le procédé de peeling comprend de préférence des étapes additionnelles de préparation de la peau au peeling (pour améliorer l'efficacité et l'homogénéité du peeling) et/ou de soin de la peau après peeling à l'aide de compositions renfermant de plus faibles quantité de vitamine B3 et/ou de vitamine C que la composition décrite précédemment.

Les compositions utilisées dans ces étapes préliminaire et supplémentaire peuvent être appliquées matin et soir, par exemple, éventuellement en association avec une composition destinée à protéger la peau contre les effets des UV. La composition de pré-traitement peut être appliquée pendant une à quatre semaines et la composition de post-traitement pendant un jour à huit semaines, par exemple.

Le procédé de peeling ci-dessus, y compris les étapes préliminaire et supplémentaire éventuelles, peut être mis en oeuvre une seule fois ou renouvelé jusqu'à cinq fois, si nécessaire, les séances de peeling étant de préférence espacées de une à huit semaines.

L'invention sera maintenant illustrée par les exemples non limitatifs suivants. Dans ces exemples, les quantités sont indiquées en pourcentage pondéral.

### EXEMPLES

### Exemple 1 : Composition de peeling anti-rides

On prépare la composition ci-dessous de façon classique pour l'homme du métier.

| | |
|---|---|
| Niacinamide | 10 % |
| Acide ascorbique | 10 % |
| Glycérine | 10 % |
| Eau purifiée | qsp 100 % |

Cette composition peut être appliquée sous forme de peeling pour atténuer les rides et ridules faciales.

### Exemple 2 : Composition de peeling blanchissant

On prépare la composition ci-dessous de façon classique pour l'homme du métier.

| | |
|---|---|
| Niacinamide | 15 % |
| Acide ascorbique | 15 % |
| Calcium pantéthéine sulfonate | 5 % |
| Propylène glycol | 30 % |
| Eau purifiée | qsp 100 % |

Cette composition peut être appliquée sous forme de peeling pour atténuer les dyschromies (lentigos actiniques ou séniles, mélasmas).

### Exemple 3 : Composition de peeling anti-acné

On prépare la composition ci-dessous de façon classique pour l'homme du métier.

| | |
|---|---|
| Niacinamide | 12 % |
| Acide ascorbique | 12 % |
| Hydroxy propyl cellulose | 2 % |
| Ethanol | 30 % |
| Eau purifiée | qsp 100 % |

Cette composition peut être appliquée sous forme de peeling pour atténuer les marques d'acné.

## Revendications

1. Procédé cosmétique de peeling comprenant l'application topique sur la peau d'une composition adaptée à une application topique sur la peau et comprenant, dans un milieu physiologiquement acceptable : (a) de 10 à 70 % en poids d'au moins un composé choisi parmi : l'acide nicotinique, les esters d'acide nicotinique et la nicotinamide, et (b) de 10 à 70 % en poids d'au moins un composé choisi parmi : l'acide ascorbique et ses précurseurs, lesdits précurseurs étant choisis parmi le palmitate d'ascorbyle, le stéarate d'ascorbyle, l'ascorbyl phosphate de magnésium, l'ascorbyl phosphate de sodium, l'ascorbyl sulfate disodique, l'acide sulfinyl-L-ascorbique et l'acide glucopyranosyl-L-ascorbique, par rapport au poids total de la composition.

2. Procédé selon la revendication 1, **caractérisé en ce que** les esters d'acide nicotinique sont choisis parmi: les alkyl esters d'acide nicotinique et d'un alcool en C₁-C₂₀.

3. Procédé selon la revendication 2, **caractérisé en ce que** les esters d'acide nicotinique sont choisis parmi : les alcools méthylique, éthylique, butylique, hexylique, octylique, décylique, dodécylique et myristylique.

4. Procédé selon l'une quelconque des revendications 1 à 3, **caractérisé en ce que** la quantité de composé (a) représente de 10 à 50 % du poids total de la composition.

5. Procédé selon l'une quelconque des revendications 1 à 4, **caractérisé en ce que** la quantité de composé (b) représente de 10 à 50 % du poids total de la composition.

6. Procédé selon l'une quelconque des revendications 1 à 5, **caractérisé en ce que** la composition ne contient pas d'acide rétinoïque ni d'acide glycolique ni de résorcinol, ni d'acide pyruvique ni d'acide salicylique ni de chitosane.

7. Procédé selon l'une quelconque des revendications 1 à 6, **caractérisé en ce qu'**il comprend les étapes consistant à : (a) appliquer topiquement sur une zone de peau humaine une composition telle que définie dans l'une quelconque des revendications 1 à 6, (b) laisser la composition au contact de la peau pendant une durée comprise entre 5 mn et 6 heures, et (c) éliminer la composition par rinçage.

8. Procédé selon la revendication 7, **caractérisé en ce que** la composition est laissée au contact de la peau, dans l'étape (b), pendant une durée comprise entre 5 mn et 30 mn.

9. Procédé selon l'une quelconque des revendications 1 à 8, **caractérisé en ce que** la composition est appliquée sur le visage et/ou le cou et/ou le décolleté et/ou les mains et/ou le dos.

10. Utilisation d'une composition telle que définie dans l'une quelconque des revendications 1 à 6 pour fabriquer une préparation dermatologique destinée à réaliser un peeling chimique superficiel par application topique sur la peau, en vue d'atténuer les rides et ridules et/ou les taches pigmentaires et/ou les cicatrices.

11. Utilisation selon la revendication 10, **caractérisée en ce que** les cicatrices sont des marques d'acné ou de varicelle.

12. Utilisation selon la revendication 10, **caractérisée en ce que** la composition est appliquée sur des personnes présentant de l'acné et/ou des rides et/ou des cicatrices et/ou des défauts de pigmentation.

## Claims

1. Cosmetic peeling method comprising the topical application, to the skin, of a composition suitable for topical application to the skin and comprising, in a physiologically acceptable medium: (a) from 10 to 70% by weight of at least one compound chosen from: nicotinic acid, nicotinic acid esters and nicotinamide, and (b) from 10 to 70% by weight of at least one compound chosen from: ascorbic acid and its precursors, the said precursors being chosen from ascorbyl palmitate, ascorbyl stearate, magnesium ascorbyl phosphate, sodium ascorbyl phosphate, disodium ascorbyl sulphate, sulphinyl-L-ascorbic acid and glucopyranosyl-L-ascorbic acid, relative to the total weight of the composition.

2. Method according to Claim 1, **characterized in that** the nicotinic acid esters are chosen from: alkyl esters of nicotinic acid and a C₁-C₂₀ alcohol.

3. Method according to Claim 2, **characterized in that** the nicotinic acid esters are chosen from: methyl, ethyl, butyl, hexyl, octyl, decyl, dodecyl and myristyl alcohols.

4. Method according to any one of Claims 1 to 3, **characterized in that** the quantity' of compound (a) represents from 10 to 50% of the total weight of the composition.

5. Method according to any one of Claims 1 to 4, **characterized in that** the quantity of compound (b) represents from 10 to 50% of the total weight of the composition.

6. Method according to any one of Claims 1 to 5, **characterized in that** the composition does not contain retinoic acid or glycolic acid or resorcinol, or pyruvic acid or salicylic acid or chitosan.

7. Method according to any one of Claims 1 to 6, **characterized in that** it comprises the steps consisting in:
(a) topically applying, to an area of human skin, a composition as defined in any one of Claims 1 to 6,
(b) leaving the composition in contact with the skin for a period of between 5 min and 6 hours, and
(c) removing the composition by rinsing.

8. Method according to Claim 7, **characterized in that** the composition is left in contact with the skin, in step (b), for a period of between 5 min and 30 min.

9. Method according to any one of Claims 1 to 8, **characterized in that** the composition is applied to the face and/or the neck and/or the neck and shoulders and/or the hands and/or the back.

10. Use of a composition as defined in any one of Claims 1 to 6, for the manufacture of a dermatological preparation intended for performing a superficial chemical peeling by topical application to the skin, in order to attenuate wrinkles and fine lines and/or pigmented spots and/or scars.

11. Use according to Claim 10, **characterized in that** the scars are acne or varicella marks.

12. Use according to Claim 10, **characterized in that** the composition is applied to people with acne and/or wrinkles and/or scars and/or pigmentation defects.

## Patentansprüche

1. Kosmetisches Peelingverfahren umfassend das topische Auftragen einer Zusammensetzung auf die Haut, die zum topischen Auftragen auf die Haut geeignet ist und, in einem physiologisch verträglichen Medium, folgendes umfasst: (a) 10 bis 70 Gew.-% von mindestens einer Verbindung, ausgewählt aus: Nikotinsäure, Nikotinsäureestern und Nikotinamid, und (b) 10 bis 70 Gew.-% von mindestens einer Verbindung, ausgewählt aus: Ascorbinsäure und ihren Vorläufern, wobei die Vorläufer aus Ascorbylpalmitat, Ascorbylstearat, Magnesiumascorbylphosphat, Natriumascorbylphosphat, Dinatriumascorbylsulfat, Sulfinyl-L-ascorbinsäure und Glucopyranosyl-L-ascorbinsäure ausgewählt sind, bezogen auf das Gesamtgewicht der Zusammensetzung.

2. Verfahren nach Anspruch 1, **dadurch gekennzeichnet, dass** die Nikotinsäureester ausgewählt sind aus: den Alkylestern von Nikotinsäure und einem C₁-C₂₀-Alkohol,

3. Verfahren nach Anspruch 2, **dadurch gekennzeichnet, dass** die Nikotinsäureester ausgewählt sind aus: Methyl-, Ethyl-, Butyl-, Hexyl-, Octyl-, Decyl-, Dodecyl- und Myristylalkoholen.

4. Verfahren nach einem der Ansprüche 1 bis 3, **dadurch gekennzeichnet, dass** die Menge von Verbindung (a) 10 bis 50 % des Gesamtgewichts der Zusammensetzung darstellt.

5. Verfahren nach einem der Ansprüche 1 bis 4, **dadurch gekennzeichnet, dass** die Menge von Verbindung (b) 10 bis 50 % des Gesamtgewichts der Zusammensetzung darstellt.

6. Verfahren nach einem der Ansprüche 1 bis 5. **dadurch gekennzeichnet, dass** die Zusammensetzung weder Rotillsäure, Glykolsäure, Resorcin, Pyruvinsäure, Salicylsäure noch Chitosan enthält.

7. Verfahren nach einem der Ansprüche 1 bis 6, **dadurch gekennzeichnet, dass** es die Schritte umfasst, die bestehen aus: (a) dem topischen Auftragen einer Zusammensetzung wie nach einem der Ansprüche 1 bis 6 definiert auf eine Zone menschlicher Haut, (b) In-Kontakt-Lassen der Zusammensetzung mit der Haut während einer Dauer von 5 min bis 6 Stunden, und (c) Beseitigen der Zusammensetzung durch Abwaschen.

8. Verfahren nach Anspruch 7, **dadurch gekennzeichnet, dass** die Zusammensetzung in Schritt (b) während einer Dauer von 5 min bis 30 min in Kontakt gelassen wird.

9. Verfahren nach einem der Ansprüche 1 bis 8, **dadurch gekennzeichnet, dass** die Zusammensetzung auf Gesicht und/oder Hals und/oder Dekolleté und/oder Hände und/oder Rücken aufgetragen wird.

10. Verwendung einer Zusammensetzung wie nach einem der Ansprüche 1 bis 6 definiert zur Herstellung eines dermatologischen Präparats, das zur Durchführung eines oberflächlichen chemischen Peelings durch topisches Auftragen auf die Haut bestimmt ist, zur Abschwächung von Falten und Fältchen und/oder von Pigmentflecken und/oder von Narben.

11. Verwendung nach Anspruch 10, **dadurch gekennzeichnet, dass** die Narben Akne- oder Windpocken-Spuren sind.

12. Verwendung nach Anspruch 10, **dadurch gekennzeichnet, dass** die Zusammensetzung auf Personen aufgetragen wird, die Akne und/oder Falten und/oder Narben und/oder Pigmentfehler aufweisen.
